# EUROPEAN PATENT SPECIFICATION

(11) **EP 1 874 377 B1**
(45) Date of publication and mention of the grant of the patent: **12.11.2014**
(21) Application number: 05739392.8
(22) Date of filing: 25.04.2005
(51) Int. Cl.: A61M 3/02, B05B 11/04

(54) **TREATMENT NOZZLE**
BEHANDLUNGSDÜSE
BUSE DE TRAITEMENT

(43) Date of publication of application: 09.01.2008
(73) Proprietor: Dolhay Klinika Egészségügyi KFT., 1037 Budapest (HU)
(72) Inventor: DOLHAY, Balázs, H-1037 Budapest (HU); DOLHAY, Gábor, H-2083 Solymár (HU)
(74) Representative: Mészarosné Donusz, Katalin
(86) International application number: PCT/HU2005/000041
(87) International publication number: WO 2006/114653

(56) References cited:
- EP-A- 0 177 456
- EP-A- 0 500 249
- WO-A-02/26294
- GB-A- 238 038
- US-A- 5 190 190
- US-A- 5 496 290
- US-B1- 6 293 928
- US-B1- 6 334 551

## Description

### Technical field

The object of the invention is a treatment nozzle, which is a treatment device suitable for treating body cavities and be applied particularly advantageously for medical and hygienic treatments, primarily for gynaecological purposes.

### Background art

Treatment devices for treating body cavities consisting of a treatment nozzle and a container made of resilient material connected thereto, for instance vaginal irrigators, are well known and are used extensively. Treatment fluid can be dispensed from such devices into the body cavity by squeezing on the treatment container, while fluids held in the body cavity can be removed by exploiting the suction effect of the treatment container. It is not possible to completely discharge the treatment fluid from such devices by a single compression of the container, and before squeezing the container again it must be allowed to expand so that air can enter it. During this operation the treatment container and the nozzle gets contaminated by the infectious medium held in the body cavity. Expended treatment devices thus become hazardous waste, and full external-internal cleansing and disinfection of the device is needed before it can be used again.

The above problem is circumvented by single-use devices, but treatment applying such devices is more expensive and the used-up devices generate significant environmental load.

Several solutions have been proposed for preventing the treatment devices from getting contaminated during use. Patent specification GB 1356410 discloses an irrigator with a nozzle that comprises a ball. After injection of the fluid has stopped, actuated by the initial reflow of the fluid the ball is seated on a proximal seat disposed inside the nozzle and thus prevents further reflowing and the contamination of the treatment container. The specification with publication number WO02/26294 discloses a vaginal irrigator consisting of a treatment container and a treatment nozzle, with two unidirectional valves being disposable in a connecting element located between the container and the nozzle. One of the valves prevents the reflow of fluid from the treatment nozzle, while the other one provides for letting air into the treatment container.

US 6334551B1 and EP500249A1 disclose further liquid dispensers with nozzles provided with valves.

A common drawback of the solutions described above is that the one-way valve applied for preventing reflow is actuated by the fluid actually reflowing into the treatment nozzle, and thus contamination of the inner space of the container is not prevented. Because of that the treatment nozzle is unsuitable for repeated use, or it has to be cleansed and disinfected before it can be used once again. Disinfection of such a device can be extremely painful, especially in the valves area.

Therefore it is needed to provide a simple treatment nozzle which can be cleansed and disinfected more simply and easily.

### Disclosure of Invention

The aim of the present invention is eliminating the above disadvantages and providing a treatment nozzle that prevents recontamination by fluids discharged from the body cavity.

The inventive aim is realised by a treatment nozzle as claimed.

The development of the inventive treatment nozzle has been motivated by the following recognitions:
A valve fully preventing the inside of the treatment nozzle from being contaminated can be realised in case the moving sealing element of the valve is disposed on the side of the contaminating medium, that is, outside the treatment device. Such sealing element keeps biologically hazardous materials outside the treatment device and does not allow biologically hazardous materials to enter the device. The moving sealing element will stop the flow of the medium through the valve opening until the pressure of the outside medium is greater or equal to the pressure inside. If we would like to discharge the medium from the treatment device, we simply increase the pressure of the medium, for instance by squeezing the treatment container connected to the device. The increased inner pressure will temporarily remove the sealing element from the valve opening and thus the medium can freely flow out into the outside environment.

Therefore, by the configuration based on the recognition we implement a one-way valve that is closed in its resting position (i.e. when the pressure is the same inside and outside), and thus the overpressure of the inner medium is needed to open it and to keep it open. If such overpressure disappears, the valve automatically closes and will remain closed. Thus it is sufficient to cleanse and disinfect the treatment nozzle from the outside before it is used once again.

According to a further recognition, the outlet valve can be implemented simply by placing an umbrella valve from distal direction into a bore disposed on the face wall of the tubular body, and disposing the valve opening on the surface covered by the sealing element of the umbrella valve. According to a still further recognition the valve can be formed by a valve opening located on the cylindrical side wall of the tubular body, with the sealing element being formed by a resilient locking ring that covers the valve opening.

In case a pressure-balancing valve capable of conducting unidirectionally towards the treatment container is disposed on the connecting element, the treatment medium can be completely discharged from the treatment device made up of the treatment container and the treatment nozzle by repeatedly squeezing and releasing the container. According to a further recognition the pressure-balancing valve and the tubular body can be disposed eccentrically on the cylindrical connecting element and thus there is more surface room for the pressure-balancing valve. With such a configuration the outlet valve and the pressure-balancing valve can be implemented using umbrella valve parts of the same size. According to a still further recognition there can be an obtuse angle between the axis of the tubular body and the connecting element, because such angle is more advantageous with respect to the relative orientation of the axis of the vagina and the hand squeezing the treatment container. A further advantage is that the flow-out point where the treatment medium contained in the treatment device leaves the vagina is located lower than the pressure-balancing valve. Thus the used-up treatment medium flowing out of the vagina will not get sucked up into the treatment container when the pressure-balancing valve is open.

The tubular body of a preferred embodiment of the inventive treatment nozzle is attached to a cylindrically symmetrical connecting element at a tilt angle of 0-45°, preferably 15°, the tubular body being preferably attached to the connecting element eccentrically, with the valve opening disposed on the face wall and/or the pressure-balancing opening disposed on the connecting element is closed by an umbrella-shaped sealing element; and the sealing element is attached to a valve stem comprising a fastening element disposed at the portion of the vale stem that is lead through a valve bore located beside the valve opening and/or the pressure balancing opening, with the thickness of the fastening element being greater than the diameter of the valve bore, and the valve opening located on face wall is closed by a retaining pin terminating in a mushroom-shaped fastening element which retains a valve disc seated against the face wall, where the valve disc comprises a retainer hole for letting through the retaining pin.

In another preferred embodiment of the inventive treatment nozzle the valve opening disposed on the conical valve seat located on the face wall is closed by a valve disc being seated against the apex of the conical valve seat by a retaining pin terminating in a mushroom-shaped fastening element, the bottom portion of the fastening element forming a support flange, with the valve disc comprising a retainer hole for letting through the retaining pin; or the valve opening disposed on the face wall is closed by a diaphragm comprising a retainer hole for letting through a retaining pin that is seated into a retaining seat located in the middle of the face wall, with the retaining pin terminating in a briquette-shaped clamping element; and the cross-sectional shape of the retaining seat disposed on the face wall and of the retaining pin is not circular, said cross-sectional shape being preferably ellipsoidal or rectangular; and in specific cases the retaining pin is fitted with a retaining projection, preferably a self-locking projection, that can be passed through the retaining seat.

In yet another preferred embodiment of the inventive treatment nozzle the valve opening disposed on the cylindrical side wall of the tubular body is closed by a diaphragm retained by an external retainer ring comprising at least two crown projections, and the crown ring consisting of the external retainer ring and the diaphragm is implemented in specific cases as a one-piece member; or the valve opening disposed on the cylindrical side wall of the tubular body is closed by a double-collar ring that is disposed in a seat bounded by a distal transverse groove and a proximal transverse groove such that it fully occupies both the distal and proximal transverse grooves, the double-collar ring being preferably implemented as a one-piece member, and the cylindrical side wall is fitted with a recessed groove that intersects the distal transverse groove, is deeper than the distal transverse groove, with the recessed groove being preferably located in line with the valve opening, in specific cases stretching diagonally from the valve opening; and the treatment nozzle comprises, on the cylindrical side wall, at least one distal column located distally with respect to the crown ring and/or the double-collar ring and at least one proximal column located proximally with respect to the crown ring and/or the double-collar ring; and it comprises on the cylindrical side wall a retaining pin for retaining the crown ring and/or the double-collar ring and a retainer hole receiving the retaining pin.

In a further preferred embodiment of the treatment nozzle according to the invention the valve opening disposed on a support surface located between portions of the tubular body having different geometry, preferably between portions having different diameter or between the dome and cylindrical side wall of the tubular body, is closed by an aperture disc pressed against the valve opening by a spring fitted on the tubular body or by a support flange thereof; and a cylindrical neck portion, bounded by a distal support surface and a proximal support surface, is disposed below the dome of the tubular body, and a valve opening is disposed on the distal and/or the proximal support surface, with the valve opening being closed by a spring fitted on the neck portion, with the end proximate to the valve opening of said spring preferably forming an aperture disc; and the treatment nozzle comprises a cylindrical block-shaped valve disc cut out from parallel-wall flat sheet, or the inside diameter of the aperture disc is smaller than the smallest diameter of the contacting portions of the retaining pin or the neck or the support flange on the one hand and the valve disc or the aperture disc on the other; or the valve opening is disposed on a protrusion or in a recess; and the treatment nozzle comprises multiple valve openings and/or recessed grooves and/or retaining pins and/or retainer holes and/or mushroom-shaped fastening elements, and/or support flanges and/or aperture discs that are in specific cases arranged symmetrically.

### Brief description of the drawings

Preferred embodiments of the inventive treatment nozzle are explained in detail with reference to the attached drawings, where
Fig. 1 shows the sectional view of one embodiment of the treatment nozzle according to the invention,
Fig. 2 is the longitudinal section of the outlet valve of the treatment nozzle shown in Fig. 1,
Fig. 3 shows the longitudinal section of the pressure-balancing valve of the treatment nozzle shown in Fig. 1,
Fig. 4 shows a preferred embodiment of the outlet valve of the inventive treatment nozzle in sectional view,
Fig. 5 shows the longitudinal sectional view of another embodiment of the outlet valve of the inventive treatment nozzle,
Fig. 6 is the sectional view of the outlet valve of another embodiment of the treatment nozzle according to the invention,
Fig. 7 is the top view of the treatment nozzle shown in Fig. 6
Fig. 8 is the half-section view of yet another example of a treatment nozzle,
Fig. 9 shows the half section view of the tubular body of still another example of a treatment nozzle,
Fig. 10 shows the half-section of the double-collar ring of the treatment nozzle shown in Fig 8 and 9,
Fig. 11 is a half-sectional view of yet another example of the outlet valve of a treatment nozzle, and
Fig 12 shows the sectional view of the outlet valve of still another example of a treatment nozzle.

### Brief description of drawings

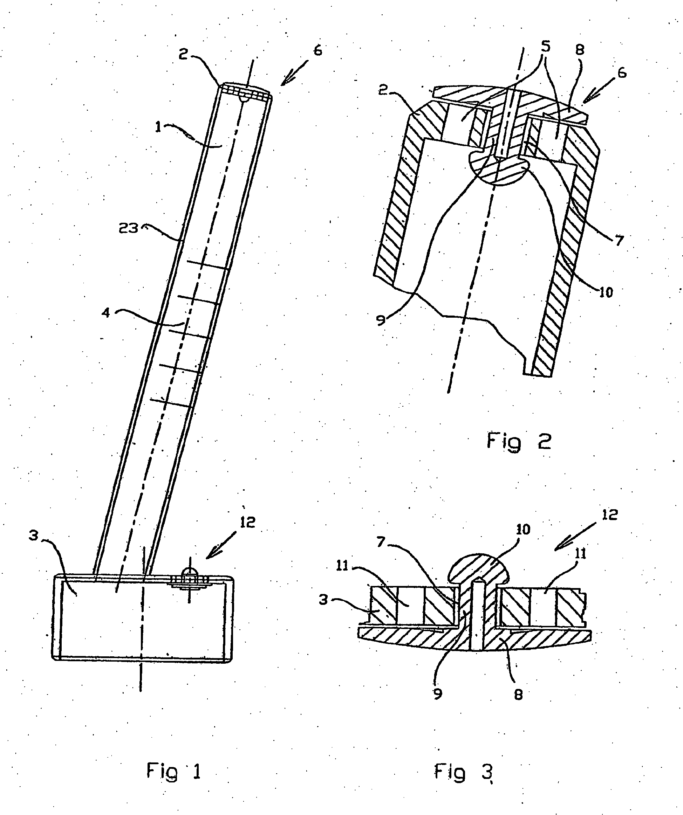

Fig. 1 shows the sectional view of the first embodiment of the inventive treatment nozzle, comprising a tubular body 1 having at its distal end a face wall 2 and terminating at its proximal end in a connecting element 3.

Fig. 1 shows the first embodiment of the inventive treatment nozzle, comprising a tubular body 1, with a face wall 2 being located at the distal end thereof. The tubular body 1 is eccentrically connected to the cylindrical connecting element 3 in such a way that there is an angle of 175° between the longitudinal axes of the tubular body 1 and the connecting element 3, that is; in other words the tubular body 1 is tilted at 15° with respect to the connecting element 3. Graduation marks 4 are disposed on the cylindrical side wall of the tubular body 1.

Figs. 1 and 2 show an outlet valve 6 consisting of valve openings 5 disposed symmetrically on the face wall 2 of the tubular body 1, an umbrella-shaped sealing element 8 inserted into the valve bore 7 from distal direction, a valve stem 9 that protrudes axial-symmetrically from the sealing element 8 and is passed through the valve bore 7, and a fastening element 10 disposed on the valve stem 9. After the shape of the sealing element 8 the outlet valve 6 can be termed an "umbrella valve" configuration.

The sealing element 8 is disposed outside the tubular body 1, while the valve stem 7 is located inside the valve bore 7, and the fastening element 10 is disposed inside the tubular body 1.

It can be discerned from Fig. 1 that in case of the embodiment shown therein a pressure balancing valve 12 is disposed in the connecting element 3 located at the end of the tubular body 1 opposite to the face wall 2. A pressure-balancing hole 11 and a valve bore 7 are disposed in the connecting element 3, with an umbrella-shaped sealing element 8 being inserted from proximal direction into the valve bore 7. The pressure-balancing valve 12 is formed by the sealing element 8, a valve stem 9 that protrudes axial-symmetrically from the sealing element 8 and is passed through the valve bore 7, and a fastening element 10 disposed on the valve stem 7. The sealing element 8 of the pressure-balancing valve 12 is disposed in the connecting element 3, while the fastening element 10 thereof is disposed externally to the connecting element 3, beside the tubular body 1. The outlet valve 6 and the pressure-balancing valve 12 have essentially identical configuration.

Now we turn to explaining the operation of the treatment nozzle shown in Figs. 1-3. The connecting element 3 of the treatment nozzle is first connected, in a manner not illustrated in the figure, to a deformable container filled with treatment substance. The pressure of the medium inside the treatment nozzle and the treatment container is initially equal to the pressure of the medium that is present outside. In this case the outlet valve 6 is closed, the sealing element 8 elastically closing the valve opening 5 from the outside. The pressure-balancing valve 12 is also elastically closed.

The depth of the introduction of the treatment nozzle into the body cavity, for instance, into the vagina, can be controlled with the help of the graduation marks 4 disposed on the cylindrical side wall of the tubular body 1. In case the internal pressure of the treatment nozzle is raised, for instance by compressing the deformable treatment container, the increased-pressure medium lifts the sealing element 8 through the valve opening 5 and the medium flows through the valve opening 5 under the elevated sealing element 8, and thus leaves the treatment nozzle. If the treatment container is held in such a way that the treatment nozzle points downwards at 15°, that is, the bottom of the treatment container is located higher than the end of the nozzle, then the treatment fluid held in the container can be discharged into the body cavity under treatment, for instance, into the vagina. If the pressure in the treatment nozzle, is reduced, for instance by ceasing to squeeze on the treatment container, under the pressure of the medium present at the distal end of the treatment nozzle, then, due to the flexibility of the container and to the pressure difference the sealing element 8 elastically closes the valve opening 5 and prevents the medium already discharged from re-entering the treatment nozzle. In case the pressure decrease in the container exceeds the pressure difference needed for actuating the pressure-balancing valve 12, the pressure-balancing valve opens and frees the pressure-balancing hole 11 and thus air can flow into the treatment container to replace the medium discharged therefrom. By squeezing on the container repeatedly, the treatment fluid can be discharged completely.

During the treatment of the body cavity neither the inside of the treatment container nor the inside of the treatment nozzle can get contaminated by the medium held in or getting discharged from the body cavity. Thus the treatment device can be cleansed after use more easily than conventional devices: it is enough to clean and disinfect the outer surface of the treatment nozzle.

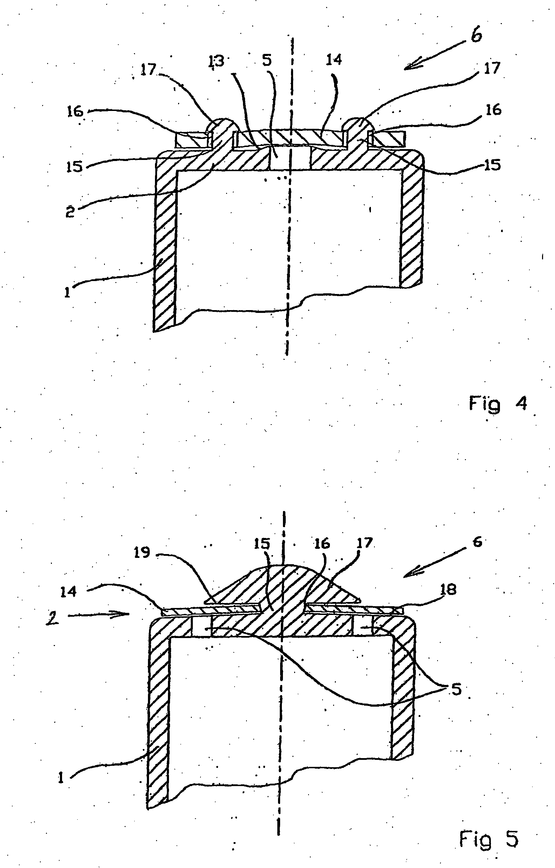

In the embodiment shown in Fig. 4 the valve opening 5 is located on a protrusion 13 disposed in the middle of the face wall 2 of the tubular body 1. The protrusion 13 is covered by a valve disc 14. The valve disc 14 is attached to the face wall 2 by retaining pins 15 that are passed through the retainer holes 16 disposed on the valve disc 14 and are terminating in a mushroom-shaped fastening element 17. The outlet valve 16 is formed by the valve openings 5 covered by the valve disc 14.

In the embodiment shown in Fig. 5 the valve openings 5 are located on the conical valve seat 18 disposed on the face wall 2 of the tubular body 1. The mushroom-shaped fastening element 17 is connected by a retaining pin 15 at the top of the conical valve seat 18. The bottom portion of the fastening element 17 forms a support flange 19 engaging a valve disc 14 that closes the valve openings 5. The outlet valve 6 is formed by the valve openings 5 covered by the valve disc 14.

In this embodiment the valve disc 14 has been cut out from parallel-wall flat sheet. The retainer hole 16 has, in its resting (undistorted) position, cylindrical shape, and its diameter is smaller than the smallest diameter of the portion of the retaining pin 15 that is contacting the valve disc 14. As it can be observed in Fig. 5, the retaining pin 15 has a distally extending conical shape. By reason of this the valve disc 14 is conically distorted and, in the case illustrated by the drawing, the inner portion of the support flange 19, supporting the valve disc 14, also assumes a conical shape.

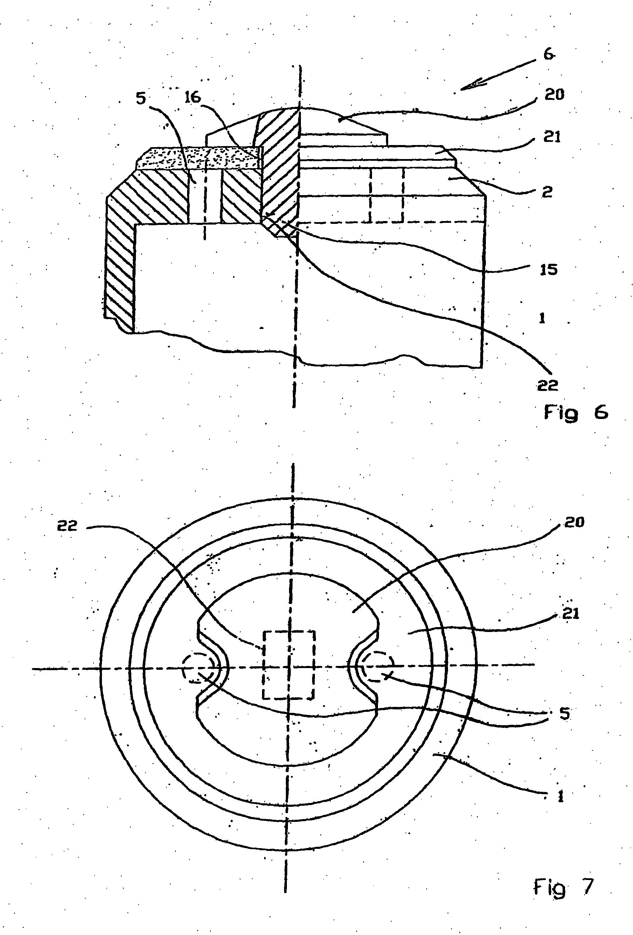

In the embodiment shown in Figs. 6 and 7 valve openings 5 are located on the face wall 2 of the tubular body 1. The valve openings 5 are closed by a diaphragm 21 pressed against the face wall 2 by a briquette-shaped clamping element 20. The retaining pin 15 disposed in the middle of the clamping element 20 is retained in the retaining seat 22 located on the face wall 2. The retaining pin 15 and retaining seat 22 have rectangular cross-sectional shape and so they can be assembled in only one position. The clamping element 20 slightly bulges out in the middle and determines the shape of the distal end of the treatment nozzle, yet it does not cover the valve openings 5. A retainer hole 16, adapted for letting through the retaining pin 15, is disposed in the middle of the diaphragm 21. The outlet valve 6 is formed by the valve openings 5 covered by the diaphragm 21.

The dimensional stability and shape retention characteristics of the valve illustrated in Figs. 6-7 ("briquette valve") are determined by the characteristics of the clamping element 20, while valve parameters are determined by the diaphragm 21 which in itself has little dimensional stability and low shape retention.

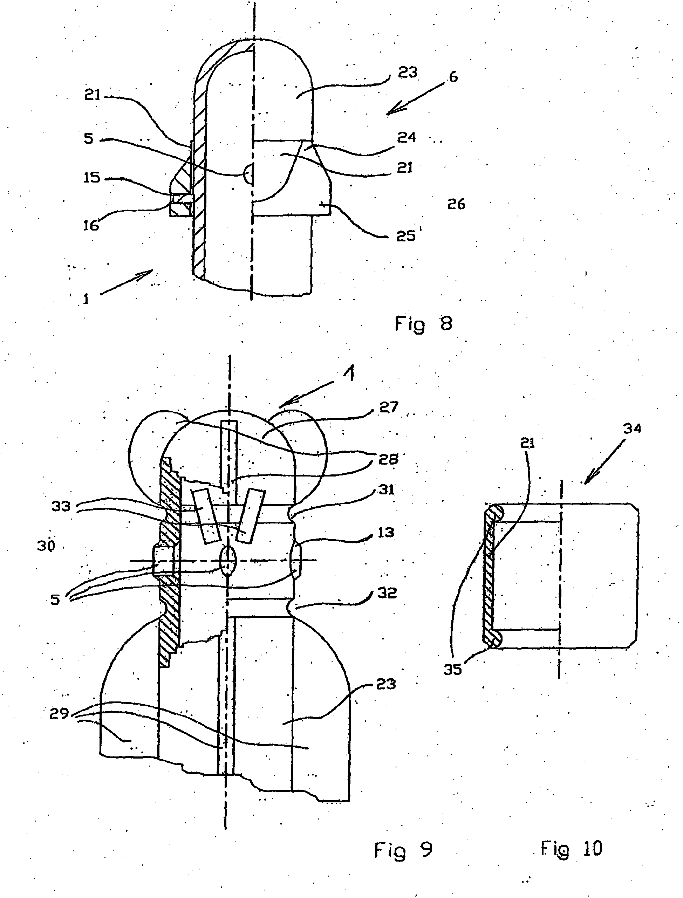

In the embodiment shown in Fig. 8 the valve opening 5 is located on the cylindrical side wall 23 of the tubular body 1, with a crown ring 26 consisting of an external retainer ring 25 comprising crown projections 24 and of a diaphragm 21 disposed under the external retainer ring 25, being distally attached thereto. Outlet valve 6 is formed by the valve opening 5 covered by the diaphragm 21 of the crown ring 26. The crown ring 26 is attached to the cylindrical side wall 23 by means of a retaining pin 15 disposed on the cylindrical side wall 23 and a retainer hole 16 that is suitably disposed on the crown ring 26 such that it receives the retaining pin 15.

The dimensional stability and shape retaining characteristics of the crown ring valve illustrated in Fig. 8 are determined by the external retainer ring 25, with the valve parameters being determined by the diaphragm 21 that in itself has little dimensional stability and low shape retention.

Fig. 9 shows the distal portion of the tubular body 1 of a treatment nozzle applying a double-collar ring. A seat 30, located between distal columns 28 extending from a dome 27 and proximal columns 29, is bounded by a distal transverse groove 31 and a proximal transverse groove 32. The valve openings 5 are formed in the middle of protrusions 13 disposed in the seat 30. Recessed grooves 33 extend distally from the valve openings 5 with said recessed grooves 33 intersecting the distal transverse groove 31 and being deeper than said distal transverse groove 31.

Fig. 10 shows a double-collar ring 34 that fully occupies both the distal transverse groove 31 and the proximal transverse groove 32, with two collars 35 being disposed at the two extremities of the ring 34. The diaphragm 21 located between the two collars 35 can be seated against protrusions 13 and can thus close the valve openings 5.

The treatment nozzle is formed by placing the double-collar ring 34 on the seat 30 in such a way that the two collars 35 are retained, respectively, in the distal transverse groove 31 and the proximal transverse groove 32. In case the internal pressure of the treatment nozzle is raised, the treatment fluid lifts up the diaphragm 21 above valve openings 5, flows into the recessed groove 33 that is deeper than the distal transverse groove 31 and thereby leaves the seat 30.

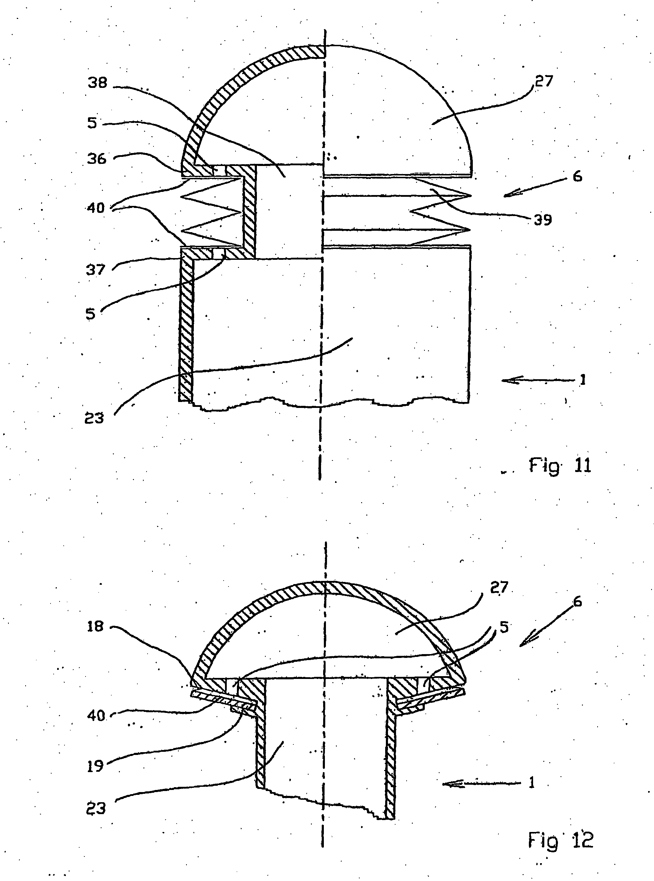

In the embodiment illustrated in Figure 11 a cylindrical neck 38 portion, being bounded by a distal support surface 36 and a proximal support surface 37, is disposed under the dome 27 of the tubular body 1. Valve openings 5 are disposed on the distal support surface 36 and on the proximal support surface 37, with the valve openings 5 being covered by aperture discs 40 formed by the uppermost and lowermost elements of a spring 39 that is mounted on neck 38. The outlet valve 6 is formed by the valve openings 5 covered by the aperture discs 40.

In the embodiment shown in Fig. 12 the valve openings are disposed on the conical valve seat 18 located under the dome 27 of the tubular body 1 which in this case has a diameter lower than the diameter of the dome 27. The valve openings 5 are kept closed by means of an aperture disc 40 supported by a support flange 19. The outlet valve 5 is formed by the valve openings 5 covered by the aperture disc 40.

In the case of this embodiment the aperture disc 40 is made of parallel-wall flat sheet. In its resting (undistorted) position both the internal and external side surfaces of the aperture disc 40 are cylindrical, with its inner diameter being smaller than the smallest diameter of the contacting portion of the tubular body 1 and the aperture disc 40. It can be observed in Fig. 12 that the portion of the tubular body 1 contacting the aperture disc 40 has a distally tapering shape. By reason of this the aperture disc 40 is conically distorted, and, in the case illustrated by the drawing, the inner portion of the support flange 19 that supports it also assumes a conical shape.

Treatment nozzles described with reference to Figs. 4-12 can be operated in the same way as the treatment nozzle applying an "umbrella valve", shown in Figs. 1-3.

The treatment nozzle according to the invention may have numerous further embodiments that are different from the ones illustrated by the drawings or that may be fitted with additional elements. For instance, the tubular body 1 can be attached to the connecting element 3 centrally, or at any tilt angle between 0-45°. The pressure-balancing valve 12 can also be fitted at a tilted position, with a tilt angle confirming to that of the tubular body 1. In Figures 1-3 the outlet valve 6 and pressure balancing valve 12 are push-in umbrella valves but the treatment nozzle can of course be implemented applying retractable umbrella valves, duckbill valves, or dome- or bell-shaped valves or a combination of these.

The valve opening 5 and/or the pressure balancing hole 11 need not necessarily be implemented as a bore, or can be realised with slanting walls and/or with a tilted orientation, and can be disposed on a protrusion 13 or in a recess. The tubular body 1 can be a smooth-wall cylinder but it may comprise at least one column and/or hollow depression.

The treatment nozzle shown in Fig. 1 may be implemented applying marks other than graduation marks 4 or without a scale. The outlet valve 6 illustrated in Fig. 4 may comprise more than two, preferably symmetrically placed mushroom-shaped fastening elements 17. The retaining pin 15 of the outlet valve 6 shown in Fig. 5 may of course have cylindrical wall, and the mushroom-shaped fastening element 17 may be produced without a support flange 19. The retaining pin 15 may be retained in the retaining seat 22 by means of adhesive binding and/or a pass-through retaining projection, preferably a self-locking retaining projection, which is thicker than the retaining pin 15. Furthermore, the retaining pin 15 and the mushroom-shaped fastening element 17 can be made so as to form a one-piece member with the face wall 2 or the tubular body 1.

The treatment nozzle according to the invention can be implemented on the support surface(s) located between portions with different geometry, preferably between portions having different diameter and/or between the dome 27 and the cylindrical side wall 23 of the tubular body 1, for instance, in a single-sided manner applying the solution illustrated in Fig. 11. If, for example, a valve opening 5 is disposed only on the proximal support surface 37, then the dome 27 may be produced without a central cavity. Furthermore, the neck 38 may be implemented by contiguous distal 36 and proximal 37 support surfaces in case both surfaces are shaped conically.

Advantageous characteristics of the inventive treatment nozzle are that it has simple configuration, it can be produced at low cost, it is suitable for multiple use, and it prevents contaminated material from entering the treatment container. It can be cleansed simply, and it is sufficient to disinfect the external surfaces only. With the inventive nozzle the treatment fluid can be completely discharged from the treatment container and thus can be utilised in its entirety. Holding the treatment device in optimal position during the treatment session is facilitated by the eccentric attachment to the connecting element and/or the tilting of the treatment nozzle.

### LIST OF REFERENCES

- 1: tubular body
- 2: face wall
- 3: connecting element
- 4: graduation marks
- 5: valve opening
- 6: outlet valve
- 7: valve bore
- 8: sealing element
- 9: valve stem
- 10: fastening element
- 11: pressure-balancing hole
- 12: pressure-balancing valve
- 13: protrusion
- 14: valve disc
- 15: retaining pin
- 16: retainer hole
- 17: fastening element
- 18: conical valve seat
- 19: support flange
- 20: clamping element
- 21: diaphragm
- 22: retaining seat
- 23: cylindrical side wall
- 24: crown protrusion
- 25: external retainer ring
- 26: crown ring
- 27: dome
- 28: distalcolumn
- 29: proximal column
- 30: seat
- 31: distal transverse groove
- 32: proximal transverse groove
- 33: recessed groove
- 34: double-collar ring
- 35: collar
- 36: distal support surface
- 37: proximal support surface
- 38: neck
- 39: spring
- 40: aperture disc

## Claims

1. Treatment nozzle, suitable for treating body cavities, of which the proximal end can be connected in specific cases to a treatment container by means of a connecting element (3), and which comprises a connecting element (3) being fitted with a one-way valve (12) for letting air into the treatment device, a tubular body having multiple valve openings (5) at its distal end, and a one-way valve (6) adapted for letting air flow out through the valve openings (5), said one-way-valve (6) being disposed on the tubular body (1), wherein the one-way valve of the treatment nozzle is disposed on the face wall (2) of the tubular body (1) and the valve openings (5) are disposed symmetrically on the face wall of the tubular body, wherein the valve openings (5) and the pressure-balancing opening (11) of the one-way valve (12) for letting air, disposed on the connecting element (3), are closed by an umbrella-shaped sealing element (8) and wherein said sealing element (8) is attached to a valve stem (9) comprising a fastening element (10) disposed at the portion of the valve stem (9) that is lead through a valve bore (7) located beside the valve openings (5) and the pressure balancing opening (11), with the thickness of the fastening element (10) being greater than the diameter of the valve bore (7).

2. The treatment nozzle according to Claim 1, **characterised by** that the tubular body (1) thereof is attached to a cylindrically symmetrical connecting element (3) at a tilt angle of 0-45°, preferably 15°, the tubular body (1) being preferably attached to the connecting element (3) eccentrically.

3. The treatment nozzle according to any of the Claims 1-2, **characterized by** that the valve openings (5) are disposed on a protrusion or in a recess.

## Patentansprüche

1. Behandlungsdüse, die zur Behandlung von Körperhöhlen geeignet ist und deren proximales Ende in bestimmten Fällen über ein Verbindungselement (3) mit einem Behandlungsbehälter verbunden werden kann und die ein Verbindungselement (3) umfasst, das mit einem Einwegventil (12) zum Einleiten von Luft in die Behandlungsvorrichtung, einem röhrenförmigen Körper mit mehreren Ventilöffnungen (5) an seinem distalen Ende, und einem Einwegventil (6) zum Ableiten von Luft durch die Ventilöffnungen (5) ausgestattet ist, wobei das Einwegventil (6) auf dem röhrenförmigen Körper (1) angeordnet ist, worin das Einwegventil der Behandlungsdüse auf der Frontwand (2) des röhrenförmigen Körpers (1) angeordnet ist und die Ventilöffnungen (5) symmetrisch auf der Frontwand des röhrenförmigen Körpers angeordnet sind, worin die Ventilöffnungen (5) und die Druckausgleichsöffnung (11) des Einwegventils (12) zum Einleiten von Luft, das auf dem Verbindungselement (3) angeordnet ist, durch ein schirmförmiges Dichtungselement (8) verschlossen werden, und worin da Dichtungselement (8) an einem Ventilschaft (9) befestigt ist, der ein Befestigungselement (10) umfasst, das an dem Teil des Ventilschafts (9) angeordnet ist, der durch eine Ventilbohrung (7) neben den Ventilöffnungen (5) und der Druckausgleichsöffnung (11) geführt wird, wobei die Dicke des Befestigungselements (10) größer als der Durchmesser der Ventilbohrung (7) ist.

2. Behandlungsdüse nach Anspruch 1, **dadurch gekennzeichnet, dass** der röhrenförmige Körper (1) der Düse an einem zylindrisch symmetrischen Verbindungselement (3) mit einem Kippwinkel von 0-45°, vorzugsweise von 15°, befestigt ist, wobei der röhrenförmige Körper (1) vorzugsweise exzentrisch an dem Verbindungselement (3) befestigt ist.

3. Behandlungsdüse nach einem der Ansprüche 1-2, **dadurch gekennzeichnet, dass** die Ventilöffnungen (5) auf einem Vorsprung oder in einer Vertiefung angeordnet sind.

## Revendications

1. Buse de traitement, appropriée pour traiter des cavités corporelles, dont l'extrémité proximale peut être connectée dans des cas spécifiques à un récipient de traitement au moyen d'un élément de connexion (3) et qui comprend un élément de connexion (3) muni d'une soupape unidirectionnelle (12) permettant d'introduire de l'air dans le dispositif de traitement, un corps tubulaire ayant plusieurs ouvertures de soupape (5) au niveau de son extrémité distale, et une soupape unidirectionnelle (6) prévue pour laisser de l'air s'écouler vers l'extérieur à travers les ouvertures de soupape (5), ladite soupape unidirectionnelle (6) étant disposée sur le corps tubulaire (1), la soupape unidirectionnelle de la buse de traitement étant disposée sur la paroi de face (2) du corps tubulaire (1) et les ouvertures de soupape (5) étant disposées symétriquement sur la paroi de face du corps tubulaire, les ouvertures de soupape (5) et l'ouverture d'équilibrage de pression (11) de la soupape unidirectionnelle (12) pour laisser passer l'air, disposées sur l'élément de connexion (3), étant fermées par un élément d'étanchéité en forme de parapluie (8) et ledit élément d'étanchéité (8) étant attaché à une tige de soupape (9) comprenant un élément d'attache (10) disposé au niveau de la portion de la tige de soupape (9) qui est guidée à travers un alésage de soupape (7) situé à côté des ouvertures de soupape (5) et de l'ouverture d'équilibrage de pression (11), l'épaisseur de l'élément d'attache (10) étant supérieure au diamètre de l'alésage de soupape (7).

2. Buse de traitement selon la revendication 1, **caractérisée en ce que** le corps tubulaire (1) de celle-ci est attaché à un élément de connexion cylindriquement symétrique (3) suivant un angle d'inclinaison de 0-45°, de préférence de 15°, le corps tubulaire (1) étant de préférence attaché à l'élément de connexion (3) de manière excentrique.

3. Buse de traitement selon l'une quelconque des revendications 1 ou 2, **caractérisée en ce que** les ouvertures de soupape (5) sont disposées sur une saillie ou dans un retrait.
